(19) Europäisches Patentamt
European Patent Office
Office européen des brevets

(11) Veröffentlichungsnummer: **0 135 894**
**B1**

(12) # EUROPÄISCHE PATENTSCHRIFT

(45) Veröffentlichungstag der Patentschrift: **23.08.89**

(51) Int. Cl.⁴: **C 07 C 127/19**, A 01 N 37/52

(21) Anmeldenummer: **84110976.2**

(22) Anmeldetag: **14.09.84**

(54) **Neue N-Carbamoylarylcarboximidsäureester, Verfahren zu ihrer Herstellung, sie enthaltende Mittel und ihre Verwendung als Schädlingsbekämpfungsmittel.**

(30) Priorität: **22.09.83 DE 3334207**

(43) Veröffentlichungstag der Anmeldung: **03.04.85 Patentblatt 85/14**

(45) Bekanntmachung des Hinweises auf die Patenterteilung: **23.08.89 Patentblatt 89/34**

(84) Benannte Vertragsstaaten: **AT BE CH DE FR GB IT LI NL SE**

(56) Entgegenhaltungen:
**EP-A-0 005 944**
**US-A-4 431 667**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Koch, Volker, Dr., Altkönigstrasse 5, D-6233 Kelkheim (Taunus) (DE)**
Erfinder: **Burghardt, Gerhard, Dr., Paulinenweg 23, D-6238 Hofheim am Taunus (DE)**

**EP 0 135 894 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Es ist bereits bekannt, daß bestimmte N-Carbamyl-2,6-difluorbenz(thio)carboximidsäureester insektizide Eigenschaften aufweisen (Europäische Patentschrift Nr. 5944). Diese besitzen jedoch Nachteile in der Anwendung wie unzureichende Wirksamkeiten.

Es wurden nun neue substituierte N-Carbamylarylcarboximidsäureester mit vorteilhaften insektiziden Eigenschaften gefunden.

Gegenstand der vorliegenden Erfindung sind daher die Verbindungen der Formel I,

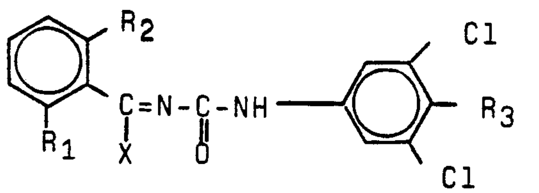

(I),

$R_1$ = H, Fluor oder Chlor,
$R_2$ = Fluor oder Chlor,
$R_3$ = ($C_1$-$C_6$)-Haloalkoxy und
X = $OR_4$, wobei $R_4$ gegebenenfalls halogeniertes ($C_1$-$C_6$)-Alkyl, ($C_5$-$C_6$)-Cycloalkyl, ($C_3$-$C_6$)-Alkenyl, ($C_3$-$C_6$)-Alkinyl oder gegebenenfalls halogeniertes Benzyl bedeutet, bedeuten.

Erfindungsgemäße Verbindungen der Formel I sind beispielsweise:

N-[N-(3,5-Dichlor-4-difluormethoxyphenyl)carbamoyl]-2,6-difluorbenzcarboximidsäureethylester
N-[N-(4-Chlordifluormethoxy-3,5-dichlorphenyl)carbamoyl]-2,6-difluorbenzcarboximidsäureethylester
N-[N-(3,5-Dichlor-4-trifluormethoxyphenyl)carbamoyl]-2,6-difluorbenzcarboximidsäureethylester
N-[N-(4-(2-Chlor-1,1,2-trifluorethoxy)-3,5-dichlorphenyl)carbamoyl]-2-fluorbenzcarboximidsäureethylester
N-[N-(4-(2-Chlor-1,1,2-trifluorethoxy)-3,5-dichlorphenyl)carbamoyl]-2-chlorbenzcarboximidsäureethylester
N-[N-(4-(2-Chlor-1,1,2-trifluorethoxy)-3,5-dichlorphenyl)carbamoyl]-2,6-difluorbenzcarboximidsäureethylester
N-[N-(4-(2-Chlor-1,1,2-trifluorethoxy)-3,5-dichlorphenyl)carbamoyl]-2-chlor-6-fluorbenzcarboximidsäureethylester
N-[N-(4-(2-Chlor-1,1,2-trifluorethoxy)-3,5-dichlorphenyl)carbamoyl]-2,6-dichlorbenzcarboximidsäureethylester
N-[N-(4-(2-Brom-1,1,2-trifluorethoxy)-3,5-dichlorphenyl)carbamoyl]-2-fluorbenzcarboximidsäureethylester
N-[N-(4-(2-Brom-1,1,2-trifluorethoxy)-3,5-dichlorphenyl)carbamoyl]-2-chlorbenzcarboximidsäureethylester
N-[N-(4-(2-Brom-1,1,2-trifluorethoxy)-3,5-dichlorphenyl)carbamoyl]-2,6-difluorbenzcarboximidsäureethylester
N-[N-(4-(2-Brom-1,1,2-trifluorethoxy)-3,5-dichlorphenyl)carbamoyl]-2-chlor-6-fluorbenzcarboximidsäureethylester
N-[N-(4-(2-Brom-1,1,2-trifluorethoxy)-3,5-dichlorphenyl)carbamoyl]-2,6-dichlorbenzcarboximidsäureethylester
N-[N-(3,5-Dichlor-4-(1,1,2,2-tetrafluorethoxy)phenyl)carbamoyl]-2-fluorbenzcarboximidsäurebenzylester
N-[N-(3,5-Dichlor-4-(1,1,2,2-tetrafluorethoxy)phenyl)carbamoyl]-2-fluorbenzcarboximidsäure-4-chlorbenzylester
N-[N-(3,5-Dichlor-4-(1,1,2,2-tetrafluorethoxy)phenyl)carbamoyl]-2-fluorbenzcarboximidsäuremethylester
N-[N-(3,5-Dichlor-4-(1,1,2,2-tetrafluorethoxy)phenyl)carbamoyl]-2-fluorbenzcarboximidsäureethylester
N-[N-(3,5-Dichlor-4-(1,1,2,2-tetrafluorethoxy)phenyl)carbamoyl]-2-fluorbenzcarboximidsäure-2,2,2-trifluorethylester
N-[N-(3,5-Dichlor-4-(1,1,2,2-tetrafluorethoxy)phenyl)carbamoyl]-2-fluorbenzcarboximidsäure-2,2,2-trichlorethylester
N-[N-(3,5-Dichlor-4-(1,1,2,2-tetrafluorethoxy)phenyl)carbamoyl]-2-chlorbenzcarboximidsäureethylester
N-[N-(3,5-Dichlor-4-(1,1,2,2-tetrafluorethoxy)phenyl)carbamoyl]-2,6-difluorbenzcarboximidsäureethylester
N-[N-(3,5-Dichlor-4-(1,1,2,2-tetrafluorethoxy)phenyl)carbamoyl]-2-chlor-6-fluorbenzcarboximidsäureethylester
N-[N-(3,5-Dichlor-4-(1,1,2,2-tetrafluorethoxy)phenyl)carbamoyl]-2,6-dichlorbenzcarboximidsäureethylester
N-[N-(3,5-Dichlor-4,(1,1,2,2-tetrafluorethoxy)phenyl)carbamoyl]-2-fluorbenzcarboximidsäure-1-propylester
N-[N-(3,5-Dichlor-4-(1,1,2,2-tetrafluorethoxy)phenyl)carbamoyl]-2-fluorbenzcarboximidsäureallylester
N-[N-(3,5-Dichlor-4-(1,1,2,2-tetrafluorethoxy)phenyl)carbamoyl]-2-fluorbenzcarboximidsäurepropargylester
N-[N-(3,5-Dichlor-4-(1,1,2,2-tetrafluorethoxy)phenyl)carbamoyl]-2-fluorbenzcarboximidsäure-1-butylester
N-[N-(3,5-Dichlor-4-(1,1,2,2-tetrafluorethoxy)phenyl)carbamoyl]-2-fluorbenzcarboximidsäure-1-hexylester
N-[N-(3,5-Dichlor-4-(1,1,2,2-tetrafluorethoxy)phenyl)carbamoyl]-2-fluorbenzcarboximidsäurecyclohexylester
N-[N-(4-(2,2-Dibrom-1,1-difluorethoxy)-3,5-dichlorphenyl)carbamoyl]-2,6-difluorcarboximidsäureethylester
N-[N-(4-(2,2-Dichlor-1,1-difluorethoxy)-3,5-dichlorphenyl)carbamoyl]-2,6-difluorbenzcarboximidsäureethylester
N-[N-(4-(2-Chlor-1,1-difluorethoxy)-3,5-dichlorphenyl)carbamoyl]-2,6-difluorbenzcarboximidsäureethylester

N-[N-(3,5-Dichlor-4-pentafluorethoxyphenyl)carbamoyl]-2,6-difluorbenzcarboximidsäureethylester
N-[N-(3,5-Dichlor-4-(1,2-dichlor-1,2-difluorethoxy)phenyl)carbamoyl]-2,6-difluorbenzcarboximidsäureethylester
N-[N-(3,5-Dichlor-4-(1,1,2,3,3,3-hexafluor-1-propoxy)phenyl)carbamoyl]-2-fluorbenzcarboximidsäureethylester
N-[N-(3,5-Dichlor-4-(1,1,2,3,3,3-hexafluor-1-propoxy)phenyl)carbamoyl]-2-chlorbenzcarboximidsäureethylester
N-[N-(3,5-Dichlor-4-(1,1,2,3,3,3-hexafluor-1-propoxy)phenyl)carbamoyl]-2,6-difluorbenzcarboximidsäureethylester
N-[N-(3,5-Dichlor-4-(1,1,2,3,3,3-hexafluor-1-propoxy)phenyl)carbamoyl]-2-chlor-6-fluorbenzcarboximidsäureethylester
N-[N-(3,5-Dichlor-4-(1,1,2,3,3,3-hexafluor-1-propoxy)phenyl)carbamoyl]-2,6-dichlorbenzcarboximidsäureethylester
N-[N-(3,5-Dichlor-4-(1,1,2,3,3,4,4,4-octafluor-1-butyloxy)phenyl)carbamoyl]-2,6-difluorbenzcarboximidsäureethylester
N-[N-(3,5-Dichlor-4-(2,2,2-trifluorethoxy)phenyl)carbamoyl]-2-fluorbenzcarboximidsäureethylester
N-[N-(3,5-Dichlor-4-(2,2,2-trifluorethoxy)phenyl)carbamoyl]-2-chlorbenzcarboximidsäureethylester
N-[N-(3,5-Dichlor-4-(2,2,2-trifluorethoxy)phenyl)carbamoyl]-2,6-difluorbenzcarboximidsäureethylester
N-[N-(3,5-Dichlor-4-(2,2,3,3-tetrafluor-1-propoxy)phenyl)carbamoyl]-2-fluorbenzcarboximidsäureethylester
N-[N-(3,5-Dichlor-4-(2,2,3,3-tetrafluor-1-propoxy)phenyl)carbamoyl]-2-chlorbenzcarboximidsäureethylester
N-[N-(3,5-Dichlor-4-(2,2,3,3-tetrafluor-1-propoxy)phenyl)carbamoyl]-2,6-difluorbenzcarboximidsäureethylester
N-[N-(3,5-Dichlor-4-(2,2,3,4,4,4-hexafluor-1-butyloxy)phenyl)carbamoyl]-2,6-difluorbenzcarboximidsäureethylester
N-[N-(3,5-Dichlor-4-(2,2,3,3,4,4,4-heptafluor-1-butyloxy)phenyl)carbamoyl]-2,6-difluorbenzcarboximidsäureethylester
N-[N-(3,5-Dichlor-4-(2,2,3,4,4,5,5,5-octafluor-1-pentyloxy)phenyl)carbamoyl]-2,6-difluorbenzcarboximidsäureethylester

Gegenstand der vorliegenden Erfindung sind auch Verfahren zur Herstellung der Verbindungen der Formel (I), dadurch gekennzeichnet, daß man

a) substituierte Arylcarboximidsäureester der Formel (II)

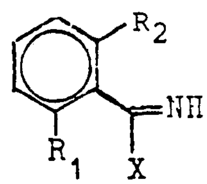

(II),

in welcher $R_1$ und $R_2$ und X die Bedeutungen wie in Formel (I) haben, mit Verbindungen der Formel (III)

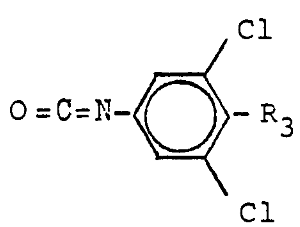

(III)

oder mit Verbindungen der Formel (IV)

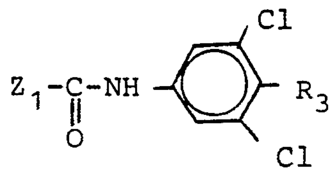

(IV),

in welchen $R_3$ die Bedeutungen wie in Formel (I) hat und $Z_1$ eine basische Abgangsgruppe außer Halogen wie beispielsweise ($C_1$-$C_3$)-Alkoxy, Phenoxy, die halogeniert sein können, Imidazol oder Triazol bedeutet, umsetzt, oder

b) substituierte Arylcarboximidsäureester-Derivate der Formel (V)

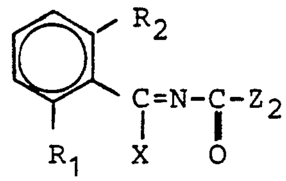

(V),

in welcher $R_1$, $R_2$ und X die Bedeutungen wie in Formel (I) haben und $Z_2$ die Bedeutung von $Z_1$ besitzt und zusätzlich für Halogen steht,

mit substituierten Anilinen der Formel (VI)

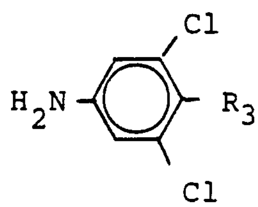

(VI),

in welcher $R_3$ die Bedeutung wie in Formel (I) hat, umsetzt

oder

c) substituierte 1,3,5-Oxadiazinderivate dar Formel (VII)

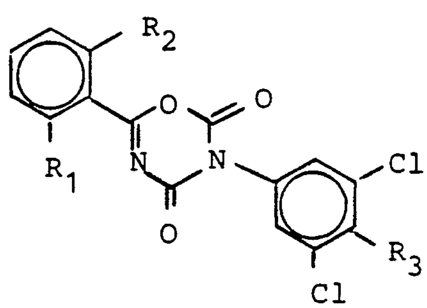

(VII),

in welcher $R_1$ bis $R_3$ die Bedeutungen wie in Formel (I) haben,

mit substituierten Hydroxyverbindungen der Formel (VIII)

$R_4OH$ (VIII),

in welcher $R_4$ die Bedeutung wie in Formel I hat, umsetzt.

Die als Ausgangsverbindungen beim Herstellungsverfahren

a) zu verwendenden Arylcarboximidsäureester der Formel (II) sind zum Teil neu und können nach an sich literaturbekannten Verfahren hergestellt werden:

1. Durch sauer oder basisch katalysierte Addition von Verbindungen der Formel (VIII) an die substituierten Nitrile der Formel (X), in welcher $R_1$ und $R_2$ die oben angegebene Bedeutung haben.

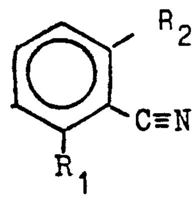

(X)

(s. R. Rogers, Chem. Rev. 61, 179 (1961))

2. Durch Umesterung von leicht zugänglichen Benzcarboximidsäureestern der Formel (II) mit Verbindungen der Formel (VIII), in denen $R_1$ bis $R_4$ die oben angeführte Bedeutung haben (R. J. Kauffmann, J. Am. Chem. Soc. 45, 1744 (1923))

3. Durch O-Alkylierung von substituierten Amiden der Formel (XI), in welcher $R_1$ und $R_2$ die oben angeführte Bedeutung haben

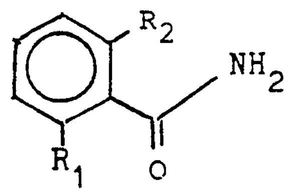

(XI)

(M. Matsui, Brit. Chem. Abstract 98, 695 (1910) und H. Meerwein, J. Prakt. Chem. 154, 154 (1940)).

Die als Ausgangsverbindungen beim Herstellungsverfahren a) zu verwendenden Verbindungen der Formel (III) und (IV) sind entweder bekannt oder können analog nach literaturbekannten Verfahren hergestellt werden (Houben-Weyl, Band VIII, 1952, Houben-Weyl, Band E 4, 1983).

Die als Ausgangsverbindungen beim Herstellungsverfahren b) zu verwendenden substituierten Arylcarboximidoester-Derivate der Formel (V) sind in an sich bekannter Weise aus Verbindungen der Formel (II) zugänglich (Houben-Weyl, Band E 4, 1983). Die für Weg b) benötigten Aniline der Formel (VI) sind entweder bekannt oder können nach an sich literaturbekannten Verfahren hergestellt werden (Houben-Weyl, Band XI/1 (1957)).

Die als Ausgangsverbindungen beim Herstellungsverfahren c) zu verwendenden 1,3,5-Oxadiazin-Derivate der Formel (VII) sind nach an sich literaturbekannten Verfahren erhältlich (US-Patent 4 150 158).

Die genannte Verfahrensvariante a) zur Herstellung von N-Aminocarbonylarylbenzcarboximidsäureestern der Formel (I) wird mit oder vorzugsweise ohne Verdünnungsmittel durchgeführt. Als Verdünnungsmittel eignen sich praktisch alle aprotischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methyl-ethyl-, Methylisopropyl- und Methylisobutylketon, Ester, wie Essigsäuremethylester und -ethylester, Nitrile, wie z. B. Acetonitril und Propionitril, Amide, wie z. B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperatur liegt im allgemeinen zwischen -10°C und +150°C, vorzugsweise zwischen +10°C und 105°C.

Die Ausgangsstoffe werden gewöhnlich in äquimolaren Mengen eingesetzt. Ein Überschuß der einen oder anderen Reaktionskomponente ist jedoch möglich.

Bei der Verfahrensvariante b) wird die Umsetzung vorzugsweise in einem Verdünnungsmittel vorgenommen. Geeignet sind alle neutralen, nicht nucleophilen organischen Lösungsmittel, bevorzugt jedoch die beim Verfahren a) genannten.

Die Reaktionstemperatur liegt im allgemeinen zwischen -10°C und +140°C, vorzugsweise zwischen +10°C und +80°C.

Die Ausgangsstoffe werden wie beim Verfahren a) gewöhnlich in äquimolaren Mengen eingesetzt. Ein Überschuß der einen oder anderen Reaktionskomponente ist jedoch möglich.

Bei der Herstellung der Verbindungen der Formel (I) nach der Verfahrensvariante c) arbeitet man bei der Umsetzung der Verbindungen der Formel (VII) mit Komponenten (VIII) zweckmäßigerweise in einem Überschuß der Komponente (VIII).

Die Reaktionstemperatur liegt im allgemeinen zwischen +20°C und +150°C, vorzugsweise zwischen +50°C und +100°C.

Die Isolierung und gegebenenfalls Reinigung der Verbindungen der Formel (I) erfolgt nach allgemein üblichen Methoden, z. B. durch Abdampfen des Lösungsmittels (gegebenenfalls unter vermindertem Druck)

und anschließendes Umkristallisieren des Rückstands oder durch Chromatographie.

Die Verbindungen der Formel (I) werden dabei als E- oder Z-Isomere oder als E/Z-Gemische erhalten. Die E/Z-Gemische lassen sich durch an sich bekannte physikalische Prozesse, wie fraktionierte Kristallisation, trennen. Beide Isomere sind biologisch aktiv und werden daher beide von vorliegender Erfindung umfaßt.

Die Verbindungen der Formel (I) sind in den meisten organischen Lösungsmitteln gut löslich.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, ganz besonders bevorzugt zur Bekämpfung von Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Bravicornyne brassicae, Cryptomyzus ribis, Doralis fabae, Doralis pomi, Eriosoma lanigerum, Hyalopterus arundinis, Macrosiphium avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella auroantii, Aspidiotus hederae, Pseudococcus spp., Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Buceulatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochlearieae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus Assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Gegenstand der Erfindung sind auch Mittel, die die Verbindungen der Formel I neben geeigneten Formulierungshilfsmitteln enthalten.

Die erfindungsgemäßen Mittel enthalten die Wirkstoffe der Formel I, im allgemeinen zu 1 - 95 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungs- oder Inertstoff noch Netzmittel, z. B. polyoxethylierte Alkylphenole, polyoxethylierte Fettalkohole, Alkyl- oder Alkylphenol-sulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z. B.

Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z. B. Talkum, natürlichen Tonen wie Kaolin, Bentonit, Poryphillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen auf die Oberfläche von Trägerstoffen wie Sand, Kaolinite, oder von granuliertem Inertmaterial. Auch können geeignete Wirkstoffe in der für die Herstellung von Düngemittelgranulaten üblichen Weise - gewünschtenfalls in Mischung mit Düngemitteln - hergestellt werden.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Ekto- und Endoparasiten vorzugsweise von ektoparasitierenden Insekten auf dem veterinärmedizinischen Gebiet bzw. auf dem Gebiet der Tierhaltung.

Die Anwendung der erfindungsgemäßen Wirkstoffe geschieht hier in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z. B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck der Insekten abhängig und lassen sich nach den üblichen Methoden leicht ermitteln und festlegen. Die neuen Verbindungen können bei Rindern z. B. in Dosiermengen von 0,01 bis 1 mg/kg Körpergewicht eingesetzt werden.

Nachfolgende Beispiele dienen zur Erläuterung der Erfindung.

**A. Formulierungsbeispiele**

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 20 Gew.-Teile Wirkstoff mit 6 Gew.-Teilen Alkylphenolpolyglykolether (Triton X 207), 3 Gew.-Teilen Isotridecanolpolyglykolether (8 AeO) und 71 Gew.-Teilen paraffinischem Mineralöl (Siedebereich z. B. ca. 255 bis über 377°C) mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertes Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand.

| Beisp. Nr. | $R^1$ | $R^2$ | $R^3$ | X | Fp °C E/Z-Gem. |
|---|---|---|---|---|---|
| 1 | F | F | $OCHF_2$ | $OC_2H_5$ | Öl |
| 2 | H | F | $OCF_2CHClF$ | $OC_2H_5$ | 128 |

| | | | | | |
|---|---|---|---|---|---|
| 3 | H | Cl | $OCF_2CHClF$ | $OC_2H_5$ | 103 - 105 |
| 4 | F | F | $OCF_2CHClF$ | $OC_2H_5$ | 103 - 105 |
| 5 | F | Cl | $OCF_2CHClF$ | $OC_2H_5$ | 123 - 124 |
| 6 | Cl | Cl | $OCF_2CHClF$ | $OC_2H_5$ | 110 - 112 |
| 7 | H | F | $OCF_2CHBrF$ | $OC_2H_5$ | 121 - 124 |
| 8 | H | Cl | $OCF_2CHBrF$ | $OC_2H_5$ | 96 |
| 9 | F | F | $OCF_2CHBrF$ | $OC_2H_5$ | 100 - 103 |
| 10 | F | Cl | $OCF_2CHBrF$ | $OC_2H_5$ | 118 - 120 |
| 11 | Cl | Cl | $OCF_2CHBrF$ | $OC_2H_5$ | 101 - 103 |
| 12 | H | F | $OCF_2CHF_2$ | $OCH_3$ | 110 - 113 |
| 13 | H | F | $OCF_2CHF_2$ | $OC_2H_5$ | 123 - 124 |
| 14 | Cl | H | $OCF_2CHF_2$ | $OC_2H_5$ | 124 - 125 |
| 15 | F | F | $OCF_2CHF_2$ | $OC_2H_5$ | 120 - 121 |
| 16 | F | Cl | $OCF_2CHF_2$ | $OC_2H_5$ | 115 - 116 |
| 16a | Cl | Cl | $OCF_2CHF_2$ | $OC_2H_5$ | 111 - 112 |
| 17 | H | F | $OCF_2CHF_2$ | $O(CH_2)_2CH_3$ | 118 |
| 18 | H | F | $OCF_2CHF_2$ | $O(CH_2)_3CH_3$ | 109 |
| 19 | H | F | $OCF_2CHF_2$ | $O(CH_2)_5CH_3$ | 82 - 83 |
| 20 | H | F | $OCF_2CHF_2$ | O- | 131 - 133 |
| 21 | H | F | $OCF_2CHFCF_3$ | $OC_2H_5$ | 105 |
| 22 | H | Cl | $OCF_2CHFCF_3$ | $OC_2H_5$ | 103 - 105 |
| 23 | F | F | $OCF_2CHFCF_3$ | $OC_2H_5$ | 94 - 97 |
| 24 | F | Cl | $OCF_2CHFCF_3$ | $OC_2H_5$ | 103 |
| 25 | Cl | Cl | $OCF_2CHFCF_3$ | $OC_2H_5$ | 90 - 91 |
| 26 | F | F | $OCH_2CF_3$ | $OC_2H_5$ | 101 |

## C. Biologische Beispiele

### Beispiel I

Spodoptera-Test

Larven des afrikanischen Baumwollwurms (Spodoptera littoralis L III) und Petrischalen, in die eine Diät auf Agar-Basis eingefüllt wird, wurden in einer Spritzapparatur mit einer Wirkstoffzubereitung der gewünschten Konzentration behandelt. Die Larven wurden nach Antrocknen des Spritzbelages auf die Agar-Diät gesetzt.

Nach der gewünschten Zeit (L III bis Falterschlupf) wurde die Abtötung der Raupen bzw. der Schlupf der Falter in % bestimmt. Dabei bedeutet 100 %, daß alle Raupen abgetötet wurden bzw. kein Falter aus den Raupen schlüpfte.

Bei diesem Test zeigten bei einem Versuch mit einer Wirkstoffkonzentration von 0,001 % die Verbindungen Nr. 2, 3, 4, 5, 6, 7, 8, 9, 12, 13, 14, 15, 16, 21, 22, 23, 24 eine 100 % Wirkung.

### Beispiel II

Musca-Test

24 Stunden alte Hausfliegenlarven (Musca domestica) wurden in eine Fliegendiät, die vorher mit einer Wirkstoffzubereitung der gewünschten Konzentration behandelt wurde, eingebracht.

Nach der gewünschten Zeit (L I bis Fliegenschlupf) wurde die Abtötung der Larven bzw. der Schlupf der Fliegen in % bestimmt. Dabei bedeutet 100 %, daß alle Larven abgetötet wurden bzw. keine Fliege aus den Puppen schlüpfte.

Bei diesem Test zeigten bei einem Versuch mit einer Wirkstoffkonzentration von 0,01 % die Verbindungen Nr. 3, 4, 5, 8, 9, 13, 16a, 22, 23 einen Wirkungsgrad von 100 %.

### Beispiel III

Aedes-Test

Man füllte die wäßrigen Wirkstoffzubereitungen der gewünschten Konzentration in Erlenmeyerkolben und setzte anschließend 24-Stunden alte Gelbfiebermückenlarven (Aedes ägypti) in die Kolben.

Nach der gewünschten Zeit (bis zum Mückenschlupf) wurde die Abtötung der Larven bzw. der Schlupf der Mücken in % bestimmt. Dabei bedeutet 100 %, daß alle Larven abgetötet wurden bzw. keine Mücke schlüpfte.

Bei diesem Test zeigten bei einem Versuch mit einer Wirkstoffkonzentration von 0,001 % die Verbindungen Nr. 3, 4, 5, 8, 9, 12, 13, 14, 15, 16, 17, 18, 20, 22, 23, 25 eine Wirkung von 100 %.

**Patentansprüche** für die Vertragsstaaten: BE, CH, DE, FR, GB, IT, LI, NL, SE

1. Verbindungen der Formel I

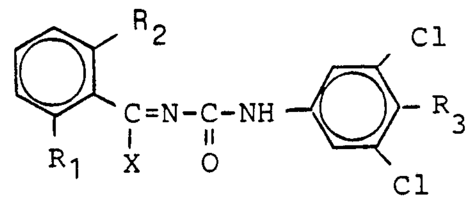

(I),

worin

$R_1$ = H, Fluor oder Chlor,

$R_2$ = Fluor oder Chlor,

$R_3$ = $(C_1\text{-}C_6)$Haloalkoxy und

X = $OR_4$, wobei $R_4$ gegebenenfalls halogeniertes $(C_1\text{-}C_6)$-Alkyl, $(C_5\text{-}C_6)$-Cycloalkyl, $(C_3\text{-}C_6)$-Alkenyl, $(C_3\text{-}C_6)$-Alkinyl oder gegebenenfalls halogeniertes Benzyl bedeutet, bedeuten.

2. Verbindungen der Formel I von Anspruch 1, dadurch gekennzeichnet, daß $R_3$ = $OCF_2CHF_2$, $OCF_2CHCl_2$, $OCF_2CHClF$, $OCF_2CHBrF$ oder $OCF_2CHFCF_3$ und X= $(C_1\text{-}C_6)$-Alkoxy bedeutet.

3. Verbindungen der Gruppe

N-[N-(3,5-Dichlor-4-(2-Chlor-1,1,2-trifluorethoxy)phenyl)carbamoyl)-2,6-difluorbenzcarboximidsäureethylester,

N-[N-(3,5-Dichlor-4-(1,1,2,2-tetrafluorethoxy)phenyl)carbamoyl]-2-fluorbenzcarboximidsäureethylester,

N-[N-(3,5-Dichlor-4-(1,1,2,2-tetrafluorethoxy)phenyl)carbamoyl]-2,6-difluorbenzcarboximidsäureethylester,

N-[N-(3,5-Dichlor-4-(1,1,2,3,3,3-hexafluorpropoxy)phenyl)carbamoyl]-2-fluorbenzcarboximidsäureethylester.

N-[N-(3,5-Dichlor-4-(1,1,2,3,3,3-hexafluorpropoxy)phenyl)carbamoyl]-2-chlor-benzcarboximidsäureethylester und

N-[N-(3,5-Dichlor-4-(1,1,2,3,3,3-hexafluorproxoxy)phenyl)carbamoyl]-2,6-difluor-benzcarboximidsäureethylester.

4. Verfahren zur Herstellung der Verbindungen der Formel (I), dadurch gekennzeichnet, daß man

a) substituierte Arylcarboximidsäureester der Formel (II)

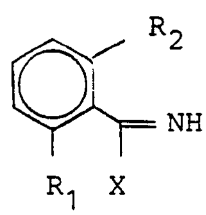

(II),

in welcher $R_1$ und $R_2$ und X die Bedeutungen wie in Formel (I) haben, mit Verbindungen der Formel (III)

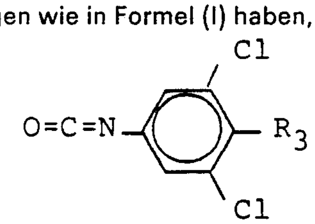

(III)

oder mit Verbindungen der Formel (IV)

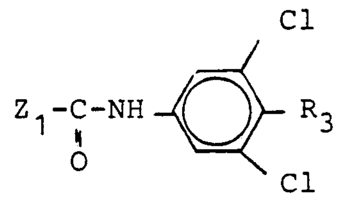

(IV),

in welchen $R_3$ die Bedeutungen wie in Formel (I) hat und $Z_1$ eine basische Abgangsgruppe außer Halogen wie beispielsweise ($C_1$-$C_3$)-Alkoxy, Phenoxy, die halogeniert sein können, Imidazol oder Triazol bedeutet, umsetzt, oder

b) substituierte Arylcarboximidsäureester-Derivate der Formel (V)

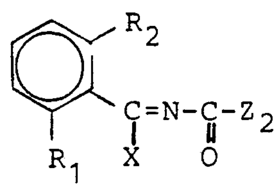

(V),

in welcher $R_1$, $R_2$ und X die Bedeutungen wie in Formel (I) haben und $Z_2$ die Bedeutung von $Z_1$ besitzt und zusätzlich für Halogen steht,

mit substituierten Anilinen der Formel (VI)

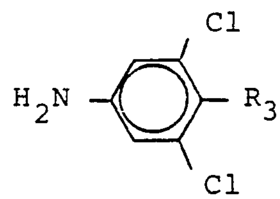

(VI),

in welcher $R_3$ die Bedeutung wie in Formel (I) hat, umsetzt oder

c) substituierte 1,3,5-Oxadiazinderivate der Formel (VII)

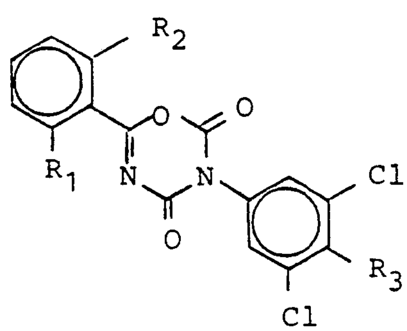

(VII)

in welcher $R_1$ bis $R_3$ die Bedeutungen wie in Formel (I) haben,

mit substituierten Hydroxyverbindungen der Formel (VIII)

$R_4OH$ (VIII)

in welcher $R_4$ die Bedeutung wie in Formel I hat, umsetzt.

5. Insektizide Mittel, dadurch gekennzeichnet, daß sie eine wirksame Menge einer Verbindung der Formel I von Anspruch 1 neben Formulierungshilfsmitteln enthalten.

6. Verfahren zur Bekämpfung von Schadinsekten, dadurch gekennzeichnet, daß man auf diese, ihre Entwicklungsstadien oder die von ihnen befallenen Pflanzen eine wirksame Menge einer Verbindung von Anspruch 1 appliziert.

7. Verwendung von Verbindungen der Formel I zur Bekämpfung von Schadinsekten.

**Patentansprüche** für den Vertragsstaat: AT

1. Verfahren zur Herstellung von Verbindungen der Formel I

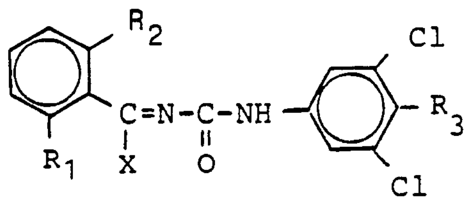

(I),

worin
$R_1$ = H, Fluor oder Chlor,
$R_2$ = Fluor oder Chlor,
$R_3$ = $(C_1\text{-}C_6)$-Haloalkoxy und
X = $OR_4$, wobei $R_4$ gegebenenfalls halogeniertes $(C_1\text{-}C_6)$-Alkyl, $(C_5\text{-}C_6)$-Cycloalkyl, $(C_3\text{-}C_6)$-Alkenyl, $(C_3\text{-}C_6)$Alkinyl oder gegebenenfalls halogeniertes Benzyl bedeutet, bedeuten,
dadurch gekennzeichnet, daß man
a) substituierte Arylcarboximidsäureester der Formel (II)

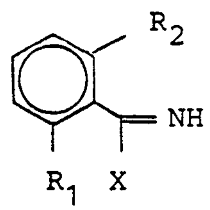

(II),

in welcher $R_1$ und $R_2$ und X die Bedeutungen wie in Formel (I) haben, mit Verbindungen der Formel (III)

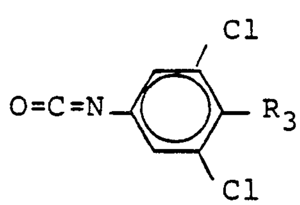

(III)

oder mit Verbindungen der Formel (IV)

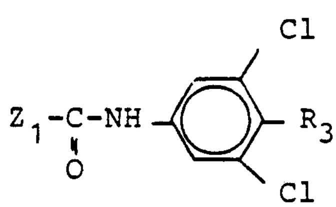

(IV),

in welchen $R_3$ die Bedeutungen wie in Formel (I) hat und $Z_1$ eine basische Abgangsgruppe außer Halogen wie beispielsweise $(C_1\text{-}C_3)$-Alkoxy, Phenoxy, die halogeniert sein können, Imidazol oder Triazol bedeutet, umsetzt, oder
b) substituierte Arylcarboximidsäureester-Derivate der Formel (V)

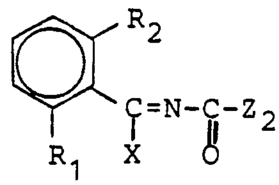

(V),

in welcher $R_1$, $R_2$ und X die Bedeutungen wie in Formel (I) haben und $Z_2$ die Bedeutung von $Z_1$ besitzt und zusätzlich für Halogen steht,

mit substituierten Anilinen der Formel (VI)

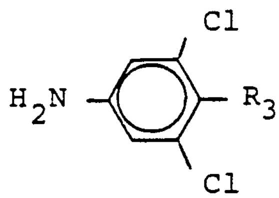

(VI),

in welcher $R_3$ die Bedeutung wie in Formel (I) hat, umsetzt oder

c) substituierte 1,3,5-Oxadiazinderivate der Formel (VII)

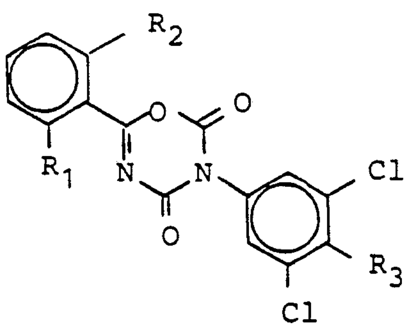

(VII),

in welcher $R_1$ bis $R_3$ die Bedeutungen wie in Formel (I) haben,

mit substituierten Hydroxyverbindungen der Formel (VIII)

$R_4OH$ (VIII),

in welcher $R_4$ die Bedeutung wie in Formel I hat, umsetzt.

2. Verfahren gemäß Anspruch 1, dadurch gekennzeichnet, daß in Formel I

$R_3$ = $OCF_2CHF_2$, $OCF_2CHCl_2$, $OCF_2CHClF$, $OCF_2CHBrF$ oder $OCF_2CHFCF_3$ und X = $(C_1\text{-}C_6)$-Alkoxy bedeutet.

3. Verfahren gemäß Anspruch 1, dadurch gekennzeichnet, daß es sich bei der Verbindung der Formel I um

N-[N-(3,5-Dichlor-4-(2-Chlor-1,1,2-trifluorethoxy)phenyl)carbamoyl)-2,6-difluorbenzcarboximidsäureethylester,

N-[N-(3,5-Dichlor-4-(1,1,2,2-tetrafluorethoxy)phenyl)carbamoyl]-2-fluorbenzcarboximidsäureethylester,

N-[N-(3,5-Dichlor-4-(1,1,2,2-tetrafluorethoxy)phenyl)carbamoyl]-2,6-difluorbenzcarboximidsäureethylester,

N-[N-(3,5-Dichlor-4-(1,1,2,3,3,3-hexafluorpropoxy)phenyl)carbamoyl]-2-fluorbenzcarboximidsäureethylester,

N-[N-(3,5-Dichlor-4-(1,1,2,3,3,3-hexafluorpropoxy)phenyl)carbamoyl]-2-chlor-benzcarboximidsäureethylester oder

N-[N-(3,5-Dichlor-4-(1,1,2,3,3,3-hexafluorproxoxy)phenyl)carbamoyl]-2,6-difluor-benzcarboximidsäureethylester handelt.

4. Insektizide Mittel, dadurch gekennzeichnet, daß sie eine wirksame Menge einer Verbindung der Formel I von Anspruch 1 neben Formulierungshilfsmitteln enthalten.

5. Verfahren zur Bekämpfung von Schadinsekten, dadurch gekennzeichnet, daß man auf diese, ihre Entwicklungsstadien oder die von ihnen befallenen Pflanzen eine wirksame Menge einer Verbindung von Anspruch 1 appliziert.

6. Verwendung von Verbindungen der Formel I zur Bekämpfung von Schadinsekten.

**Claims** for the Contracting States: BE, CH, DE, FR, GB, IT, LI, NL, SE

1. A compound of the formula I

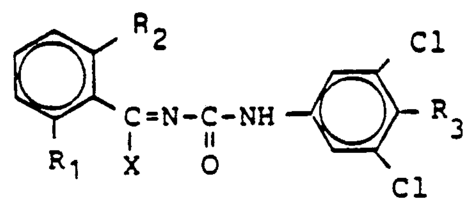

(I),

in which
$R_1$ denotes H, fluorine or chlorine,
$R_2$ denotes fluorine or chlorine,
$R_3$ denotes ($C_1$-$C_6$)-haloalkoxy and
X denotes $OR_4$ in which $R_4$ denotes optionally halogenated ($C_1$-$C_6$)-alkyl, ($C_5$-$C_6$)-cycloalkyl, ($C_3$-$C_6$)-alkenyl or ($C_3$-$C_6$)-alkynyl or optionally halogenated benzyl.

2. A process as claimed in claim 1, wherein
$R_3$ denotes $OCF_2CHF_2$, $OCF_2CHCl_2$, $OCF_2CHClF$, $OCF_2CHBrF$ or $OCF_2CHFCF_3$ and
X denotes ($C_1$-$C_6$)-alkoxy.

3. A compound of the group:
ethyl N-[N-(3,5-dichloro-4-(2-chloro-1,1,2-trifluoroethoxy)phenylcarbamoyl]-2,6-difluorobenzocarboximidate,
ethyl N-[N-(3,5-dichloro-4-(1,1,2,2-tetrafluoroethoxy)phenyl)carbamoyl]-2-fluorobenzocarboximidate,
ethyl N-[N-(3,5-dichloro-4-(1,1,2,2-tetrafluoroethoxy)phenyl)carbamoyl]-2,6-difluorobenzocarboximidate,
ethyl N-[N-(3,5-dichloro-4-(1,1,2,3,3,3-hexafluoropropoxy)phenyl)carbamoyl]-2-fluorobenzocarboximidate,
ethyl N-[N-(3,5-dichloro-4-(1,1,2,3,3,3-hexafluoropropoxy)phenyl)carbamoyl]-2-chloro-benzocarboximidate and
ethyl N-[N-(3,5-dichloro-4-(1,1,2,3,3,3-hexafluoropropoxy)phenyl)carbamoyl]-2,6-difluorobenzocarboximidate.

4. A process for the preparation of a compound of the formula I, which comprises
a) reacting a substituted arylcarboximidic acid ester of the formula II

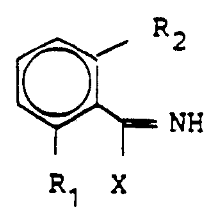

(II),

in which $R_1$ and $R_2$ and X have the same meanings as in formula I with a compound of the formula III

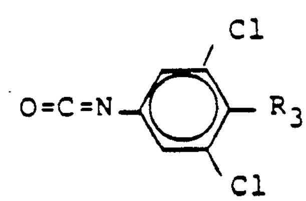

(III)

or with a compound of the formula IV

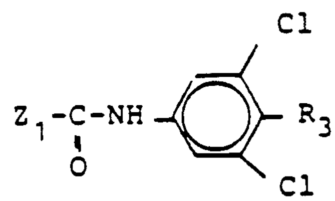

(IV),

in which $R_3$ has the same meanings as in formula I and $Z_1$ denotes a basic detachable group other than halogen, such as, for example, $(C_1-C_3)$-alkoxy, phenoxy, which can be halogenated, imidazole or triazole, or

b) reacting a substituted derivative of an aryl carboximidic acid ester of the formula V

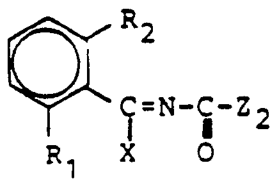

(V),

in which $R_1$, $R_2$ and X have the same meanings as in formula I and $Z_2$ has the meaning of $Z_1$ and additionally represents halogen with a substituted aniline of the formula VI

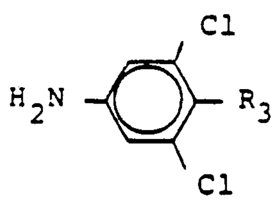

(VI),

in which $R_3$ has the same meaning as in formula I, or

c) reacting a substituted 1,3,5-oxadiazine derivative of the formula VII

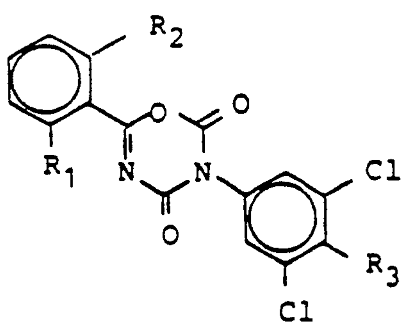

(VII)

in which $R_1$ to $R_3$ have the same meanings as in formula I with a substituted hydroxyl compound of the formula VIII

$R_4OH$ (VIII)

in which $R_4$ has the same meaning as in formula I.

5. An insecticidal agent which contains an effective amount of a compound of the formula I as claimed in claim 1 together with formulation auxiliaries.

6. A process for combating harmful insects, wherein an effective amount of a compound of claim 1 is applied to these insects, to their development stages or to the plants infested by them.

7. The use of a compound of the formula I for combating harmful insects.

**Claims** for the Contracting State: AT

1. A process for the preparation of a compound of the formula I

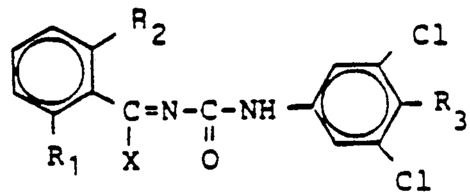

(I);

in which
$R_1$ denotes H, fluorine or chlorine,
$R_2$ denotes fluorine or chlorine,
$R_3$ denotes ($C_1$-$C_6$)-haloalkoxy and
X denotes $OR_4$ in which $R_4$ denotes optionally halogenated ($C_1$-$C_6$)-alkyl, ($C_5$-$C_6$)-cycloalkyl, ($C_3$-$C_6$)-alkenyl or ($C_3$-$C_6$)-alkynyl or optionally halogenated benzyl, which comprises,
a) reacting a substituted arylcarboximidic acid ester of the formula II

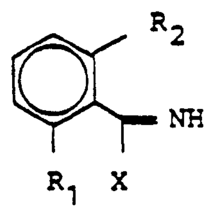

(II),

in which $R_1$ and $R_2$ and X have the same meanings as in formula I with a compound of the formula III

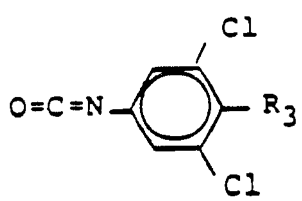

(III)

or with a compound of the formula IV

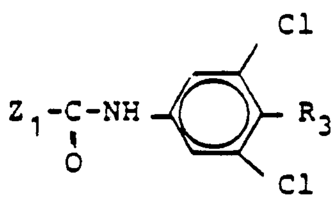

(IV),

in which $R_3$ has the same meanings as in formula I and $Z_1$ denotes a basic detachable group other than halogen, such as, for example, ($C_1$-$C_3$)-alkoxy, phenoxy, which can be halogenated, imidazole or triazole, or
b) reacting a substituted derivative of an aryl carboximidic acid ester of the formula V

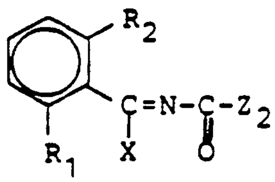

(V),

in which $R_1$, $R_2$ and X have the same meanings as in formula I and $Z_2$ has the meaning of $Z_1$ and additionally represents halogen with a substituted aniline of the formula VI

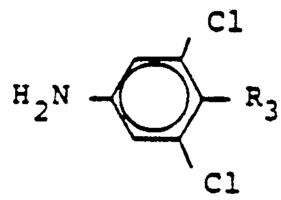

(VI),

in which $R_3$ has the same meaning as in formula I, or

c) reacting a substituted 1,3,5-oxadiazine derivative of the formula VII

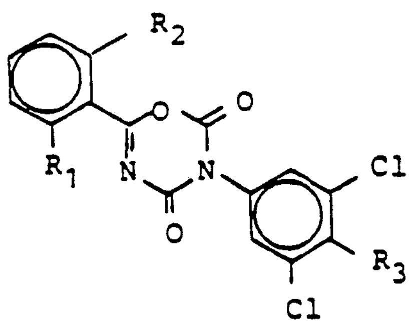

(VII),

in which $R_1$ to $R_3$ have the same meanings as in formula I with a substituted hydroxyl compound of the formula VIII

$R_4OH$ (VIII)

in which $R_4$ has the same meaning as in formula I.

2. A process as claimed in claim 1, wherein $R_3$ denotes $OCF_2CHF_2$, $OCF_2CHCl_2$, $OCF_2CHClF$, $OCF_2CHBrF$ or $DCF_2CHFCF_3$ and X denotes $(C_1-C_6)$-alkoxy.

3. A process as claimed in claim 1, wherein a compound of the formula I is used:
ethyl N-[N-(3,5-dichloro-4-(2-chloro-1,1,2-trifluoroethoxy)phenylcarbamoyl]-2,6-difluorobenzocarboximidate,
ethyl N-[N-(3,5-dichloro-4-(1,1,2,2-tetrafluoroethoxy)phenyl)carbamoyl]-2-fluorobenzocarboximidate,
ethyl N-[N-(3,5-dichloro-4-(1,1,2,2-tetrafluoroethoxy)phenyl)carbamoyl]-2,6-difluorobenzocarboximidate,
ethyl N-[N-(3,5-dichloro-4-(1,1,2,3,3,3-hexafluoropropoxy)phenyl)carbamoyl]-2-fluorobenzocarboximidate,
ethyl N-[N-(3,5-dichloro-4-(1,1,2,3,3,3-hexafluoropropoxy)phenyl)carbamoyl]-2-chloro-benzocarboximidate or
ethyl N-[N-(3,5-dichloro-4-(1,1,2,3,3,3-hexafluoropropoxy)phenyl)carbamoyl]-2,6-difluorobenzocarboximidate.

4. An insecticidal agent which contains an effective amount of a compound of the formula I as claimed in claim 1 together with formulation auxiliaries.

5. A process for combating harmful insects, wherein an effective amount of a compound of claim 1 is applied to these insects, to their development stages or to the plants infested by them.

6. The use of a compound of the formula I for combating harmful insects.

**Revendications** pour les Etats Contractants: BE, CH, DE, FR, GB, IT, LI, NL, SE

1. Composés répondant à la formule I:

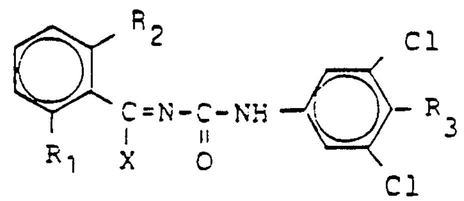

(I)

dans laquelle:

$R_1$ représente H, le fluor ou le chlore,

$R_2$ représente le fluor ou le chlore,

$R_3$ représente un radical halogéno-alcoxy en $C_1$-$C_6$ et

X représente un radical $-OR_4$ dans lequel $R_4$ représente un alkyle en $C_1$-$C_6$ éventuellement halogéné, un cycloalkyle en $C_5$ ou $C_6$, un alcényle en $C_3$-$C_6$, un alcynyle en $C_3$-$C_6$ ou un benzyle éventuellement halogéné.

2. Composés de formule I selon la revendication 1, caractérisés en ce que $R^3$ représente un radical $OCF_2CHF_2$, $OCF_2CHCl_2$, $OCF_2CHClF$, $OCF_2CHBrF$ ou $OCF_2CHFCF_3$ et X représente un alcoxy en $C_1$-$C_6$.

3. Composés de l'ensemble constitué par:

le N-[N-(dichloro-3,5 (chloro-2 trifluoro-1,1,2 éthoxy)-4 phényl)-carbamoyl] difluoro-2,6 benzène-carboximidate d'éthyle,

le N-[N-(dichloro-3,5 (tétrafluoro-1,1,2,2 éthoxy)-4 phényl)-carbamoyl] fluoro-2 benzène-carboximidate d'éthyle,

le N-[N-(dichloro-3,5 (tétrafluoro-1,1,2,2 éthoxy)-4 phényl)-carbamoyl] difluoro-2,6 benzène-carboximidate d'éthyle,

le N-[N-(dichloro-3,5 (hexafluoro-1,1,2,3,3,3 propoxy)-4 phényl)-carbamoyl] fluoro-2 benzène-carboximidate d'éthyle,

le N-[N-(dichloro-3,5 (hexafluoro-1,1,2,3,3,3 propoxy)-4 phényl)-carbamoyl] chloro-2 benzène-carboximidate d'éthyle et

le N-[N-(dichloro-3,5 (hexafluoro-1,1,2,3,3,3 propoxy)-4 phényl)-carbamoyl] difluoro-2,6 benzène-carboximidate d'éthyle.

4. Procédé pour préparer les composés de formule I, procédé caractérisé en ce que:

a) on fait réagir des esters d'acides arène-carboximidiques substitués répondant à la formule II:

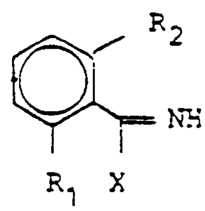

(II)

dans laquelle $R_1$, $R_2$ et X ont les significations qui leur ont été données à propos de la formule I,

avec des composés répondant à la formule III:

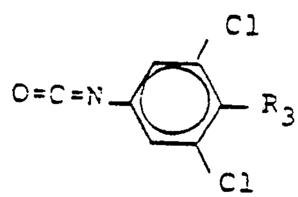

(III)

ou avec des composés répondant à la formule IV:

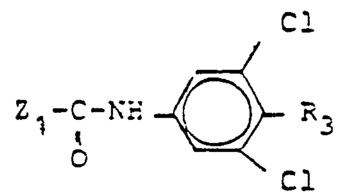

(IV),

formules dans lesquelles $R_3$ a les significations qui lui ont été données à propos de la formule I et $Z_1$ représente un radical basique éliminable autre qu'un halogène, par exemple un radical alcoxy en $C_1$-$C_3$, un radical phénoxy, lesquels peuvent être halogénés, un radical d'imidazole ou un radical de triazole,

ou

b) on fait réagir des dérivés d'esters d'acides arène-carboximidiques substitués répondant à la formule V:

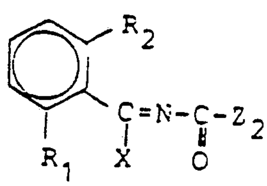

(V)

dans laquelle $R_1$, $R_2$ et X ont les significations qui leur ont été données à propos de la formule I et $Z_2$ a la signification qui a été donnée pour $Z_1$ et peut en outre représenter un halogène,

avec des anilines substituées répondant à la formule VI:

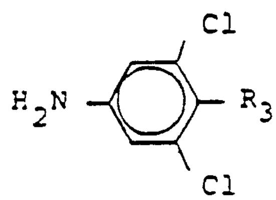

(VI)

dans laquelle:

$R_3$ a la signification qui lui a été donnée à propos de la formule I,

ou

c) on fait réagir des oxadiazines-1,3,5 substituées répondant à la formule VII:

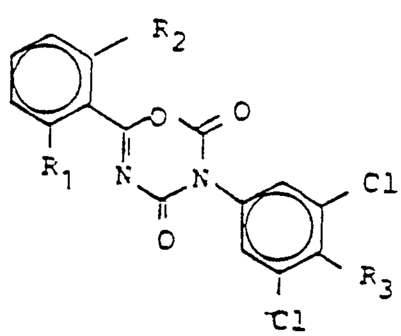

(VII)

dans laquelle:

$R_1$ à $R_3$ ont les significations qui leur ont été données à propos de la formule I,

avec des composés hydroxylés substitués répondant à la formule VIII:

$R_4OH$ (VIII)

dans laquelle:

$R_4$ a la signification qui lui a été donnée à propos de la formule I.

5. Produits insecticides caractérisés en ce qu'ils contiennent une quantité efficace d'un composé de formule

I selon la revendication 1 ainsi que des adjuvants de formulation.

6. Procédé pour combattre des insectes nuisibles, procédé caractérisé en ce qu'on applique sur ceux-ci, sur leurs stades de développement ou sur les plantes qu'ils infestent, une quantité efficace d'un composé selon la revendication 1.

7. Application de composés de formule I à la lutte contre des insectes nuisibles.

**Revendications** pour l'Etat Contractant: AT

1. Procédé de préparation de composés répondant à formule I:

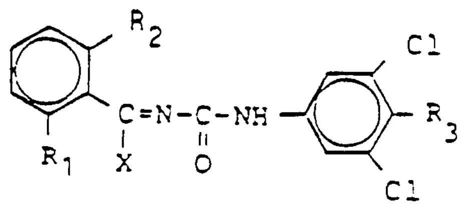

(I),

dans laquelle:

$R_1$ représente H, le fluor ou le chlore,

$R_2$ représente le fluor ou le chlore,

$R_3$ représente un radical halogéno-alcoxy en $C_1$-$C_6$ et

X représente un radical $-OR_4$ dans lequel $R_4$ représente un alkyle en $C_1$-$C_6$ éventuellement halogéné, un cycloalkyle en $C_5$ ou $C_6$, un alcényle en $C_3$-$C_6$, un alcynyle en $C_3$-$C_6$ ou un benzyle éventuellement halogéné;

procédé caractérisé en ce que:

a) On fait réagir des esters d'acides arène-carboximidiques substitués répondant à la formule II:

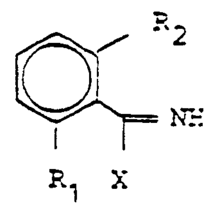

(II)

dans laquelle:

$R_1$, $R_2$ et X ont les significations qui leur ont été données à propos de la formule I,

avec des composés répondant à la formule III:

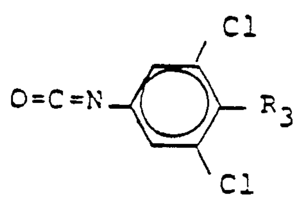

(III)

ou avec des composés répondant à la formule IV:

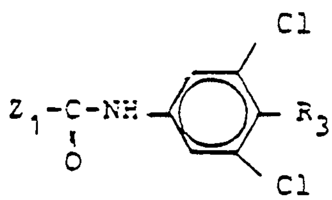

(IV),

formules dans lesquelles $R_3$ a les significations qui lui ont été données à propos de la formule I et $Z_1$ représente un radical basique éliminable autre qu'un halogène, par exemple un radical alcoxy en $C_1$-$C_3$, un radical

phénoxy, lesquels peuvent être halogénés, un radical d'imidazole ou un radical de triazole,

ou

b) on fait réagir des dérivés d'esters d'acides arène-carboximidiques substitués répondant à la formule V:

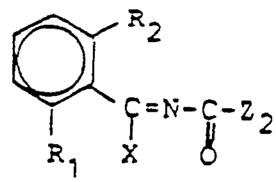

(V)

dans laquelle:

$R_1$, $R_2$ et X ont les significations qui leur ont été données à propos de la formule I et $Z_2$ a la signification qui a été donnée pour $Z_1$ et peut en outre représenter un halogène,

avec des anilines substituées répondant à la formule VI:

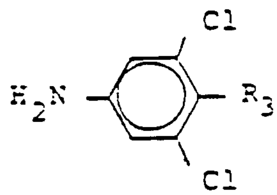

(VI)

dans laquelle:

$R_3$ a la signification qui lui a été donnée à propos de la formule I,

ou

c) on fait réagir des oxadiazines-1,3,5 substituées- répondant à la formule VII:

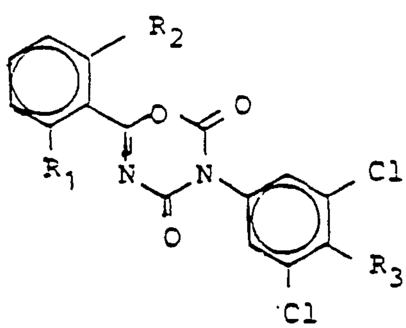

(VII)

dans laquelle:

$R_1$ à $R_3$ ont les significations qui leur ont été données à propos de la formule I,

avec des composés hydroxylés substitués répondant à la formule VIII:

$R_4OH$ (VIII)

dans laquelle:

$R_4$ a la signification qui lui a été donnée à propos de la formule I.

2. Procédé selon la revendication 1, caractérisé en ce que, dans la formule I, $R_3$ représente un radical $OCF_2CHF_2$, $OCF_2CHCl_2$, $OCF_2CHClF$, $OCF_2CHBrF$ ou $OCF_2CHFCF_3$ et X représente un alcoxy en $C_1$-$C_6$.

3. Procédé selon la revendication 1 caractérisé en ce que le composé de formule I est un composé pris dans l'ensemble constitué par:

le N-[N-(dichloro-3,5 (chloro-2 trifluoro-1,1,2 éthoxy)-4 phényl)-carbamoyl] difluoro-2,6 benzène-carboximidate d'éthyle,

le N-[N-(dichloro-3,5 (tétrafluoro-1,1,2,2 éthoxy)-4 phényl)-carbamoyl] fluoro-2 benzène-carboximidate d'éthyle,

le N-[N-(dichloro-3,5 (tétrafluoro-1,1,2,2 éthoxy)-4 phényl)-carbamoyl] difluoro-2,6 benzène-carboximidate d'éthyle,

le N-[N-(dichloro-3,5 (hexafluoro-1,1,2,3,3,3 propoxy)-4 phényl)-carbamoyl] fluoro-2 benzène-carboximidate d'éthyle,

le N-[N-(dichloro-3,5 (hexafluoro-1,1,2,3,3,3 propoxy)-4 phényl)-carbamoyl] chloro-2 benzène-carboximidate d'éthyle et

le N-[N-(dichloro-3,5 (hexafluoro-1,1,2,3,3,3 propoxy)-4 phényl)-carbamoyl] difluoro-2,6 benzène-carboximidate d'éthyle.

4. Produits insecticides caractérisés en ce qu'ils contiennent une quantité efficace d'un composé de formule I selon la revendication 1 ainsi que des adjuvants de formulation.

5. Procédé pour combattre des insectes nuisibles, procédé caractérisé en ce qu'on applique sur ceux-ci, sur leurs stades de développement ou sur les plantes qu'ils infestent, une quantité efficace d'un composé selon la revendication 1.

6. Application de composés de formule I à la lutte contre des insectes nuisibles.